# NEW EUROPEAN PATENT SPECIFICATION

(11) **EP 2 023 958 B2**
(45) Date of publication and mention of the opposition decision: **22.07.2020**
(45) Mention of the grant of the patent: 25.10.2017
(21) Application number: 07748029.1
(22) Date of filing: 18.04.2007
(51) Int. Cl.: A61K 47/18, A61K 31/465, A61K 9/00, A61K 9/12, A61K 9/20, A61K 9/48, A61K 9/68, A61K 9/70, A61P 1/04, A61P 25/16, A61P 25/28, A61P 25/34

(54) **PHARMACEUTICAL PRODUCT FOR INTRAORAL DELIVERY OF NICOTINE COMPRISING TROMETAMOL AS BUFFERING AGENT**
PHARMAZEUTISCHES PRODUKT ZUR INTRAORALEN ABGABE VON NIKOTIN MIT TROMETAMOL ALS PUFFERMITTEL
PRODUIT PHARMACEUTIQUE DESTINÉ À UNE ADMINISTRATION INTRABUCCALE DE NICOTINE COMPRENANT DU TROMÉTAMOL SERVANT D'AGENT TAMPON

(30) Priority: 16.05.2006 SE 0601089
(43) Date of publication of application: 18.02.2009
(73) Proprietor: McNeil AB, 25109 Helsingborg (SE)
(72) Inventor: STEEN, Per, 265 38 Åstorp (SE); DYMITROWICS, Darek, 211 50 Malmö (SE); HUGERTH, Andreas, 2-237 33 Bjärred (SE); WALTERMO, Åsa, 260 35 Ödåkra (SE); PÅLSSON, Magnus, 256 56 Helsingborg (SE); OLSSON, Roland, 253 76 Helsingborg (SE); NICKLASSON, Fredrik, 217 59 Malmö (SE); SCHLÜTER, Anette, 257 32 Rydebäck (SE); THYRESSON, Kristina, 226 57 Lund (SE); MODY, Seema, K., Montville, NJ 07045 (US); BOSSON, Bengt, 256 55 Helsingborg (SE); LINDELL, Katarina, 241 93 Eslöv (SE)
(74) Representative: Kirsch, Susan Edith
(86) International application number: PCT/SE2007/000364
(87) International publication number: WO 2007/133140

(56) References cited:
- EP-A2- 0 380 021
- WO-A1-01/30288
- WO-A1-02/12357
- WO-A1-02/102357
- WO-A1-03/055486
- US-A- 3 471 613
- US-A- 5 783 207
- US-A- 6 166 044
- US-A1- 2004 037 879
- US-A1- 2004 052 851
- US-A1- 2006 002 865
- "Tromethamine" In: Alsonso R Gennaro (Ed): "Remington: The Science and Practice of Pharmacy, 20th Edition", 2000, Lippincott Williams & Wilkins, Philadelphia PA, US, XP002680007, ISBN: 0683306472 pages 1266-1266, * page 1266, column 1 *
- Printout from EU Clinical Trials Register
- Clinical Study Report A6431081
- Nicorette
- McNeil previous study carbonate
- Nicotinell

## Description

### Technical Field

This invention relates to nicotine-containing pharmaceutical formulations for intraoral delivery of nicotine to a subject. The formulations comprise the buffer trometamol. Also contemplated are a method and a system for delivering nicotine as well as use and production of said formulations.

### Background of the Invention

Tobacco dependence and reduction thereof is a desirable goal. In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, and smokers characteristically display a strong tendency to relapse after having successfully stopped smoking for a time. Nicotine is the world's second most used drug, after caffeine from coffee and tea.

The main problem with tobacco smoking is its enormous implications on health. It is estimated that smoking related diseases cause some 3 - 4 million deaths per year. According to Centers for Disease Control and Prevention, cigarette smoking among adults - United States, 1995. MMWR 1997; 46:1217 -1220 around 500,000 persons in USA die each year as a result of tobacco use. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug. The incidence of smoking is still rising in many countries, especially in less developed countries.

The most advantageous thing a heavy smoker can do is to stop smoking completely or at least to his/her smoking. Experience shows, however, that most smokers find this extremely difficult since, mostly, tobacco smoking results in a dependence disorder or craving. The World Health Organization ("WHO") has in its International Classification of Disorders a diagnosis called Tobacco Dependence. Others like the American Psychiatric Association call the addiction Nicotine Dependence. It is generally accepted that these difficulties to stop smoking result from the fact that those heavy smokers are dependent on nicotine. The most important risk factors related to health are, however, substances that are formed during the combustion of tobacco, such as tar products, carbon monoxide, aldehydes, and hydrocyanic acid.

### Effects of nicotine

Nicotine is an addictive poisonous alkaloid C₅H₄NC₄H₇NCH₃, derived from the tobacco plant. Nicotine is also used as an insecticide. Approximately 40 milligrams of nicotine as a single dose may kill an adult (Merck Index). The administration of nicotine (for example, in the form of smoking a cigarette, cigar or pipe) can give a pleasurable feeling to the smoker. However, smoking has health hazards and it is, therefore, desirable to formulate an alternative way of administering nicotine in a pleasurable and harmless manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction usually lasts during the smoking time of the cigarette and for a period of time thereafter. The poisonous, toxic, carcinogenic, and addictive nature of smoking has provided strong motivation to develop methods, compositions and devices, which can be used to break the habit of smoking cigarettes.

### Nicotine replacement products

One way to reduce smoking is to provide nicotine in a form or manner other than by smoking and some products have been developed to fulfil this need. Nicotine containing formulations are currently the dominating treatments for tobacco dependence.

The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market. Several methods and means have been described for diminishing the desire of a subject to use tobacco, which comprises the step of administering to the subject nicotine or a derivative thereof as described in e g U.S. Patent Number 5,810,018 (oral nicotine-containing spray), U.S. Patent Number 5,939,100 (nicotine-containing micro spheres) and U.S. Patent Number 4,967,773 (nicotine-containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Ways of administrating nicotine by way of delivering directly into the nasal cavity by spraying is known from U.S. Patent Number 4,579,858, DE 32 41 437 and WO/93 127 64. There may be local nasal irritation, however, with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in U.S. Patent Number 5,167,242. Said means and methods address the problems associated with addiction to nicotine.

One successful product that is used as a smoking substitute and/or as a smoking cessation aid and which is based on nicotine, is the chewing gum Nicorette®. This product was one of the first nicotine replacement forms that was approved by the Food and Drug Administration (FDA) and is still one of the most used nicotine replacement products. Nicorette® chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active. Patents related to this product are e g U.S. Patent Number 3,877,468, U.S. Patent Number 3,901,248 and U.S. Patent Number 3,845,217.

### Prior art and problems thereof

WO 02/102357 discloses a coated nicotine-containing chewing gum. This gum provides improved transmucousal absorption of nicotine in the oral cavity. Thereby is achieved more of a cigarette-like sense of satisfaction and a more rapid reduction of the urge to smoke. The buffers proposed in WO 02/102357 possess off-notes, however, and one or more flavoring agents need be added to the gum in order to cover the off-note taste.

WO 03/055486 discloses a mouth spray comprising nicotine in any form for administration to the oral cavity being alkalized by buffering and/or pH regulation. Buffers such as carbonates, glycinate, phosphate, glycerophosphate or citrate of an alkali metal are suggested.

Problems with off-notes from buffers may also arise with all other nicotine-containing pharmaceutical formulations for oral delivery, such as mouth sprays, chewable tablets, tablets, lozenges, capsules, lipid-filled micro gels, oral films, and different candy-type formulations.

Thus, there is a need to ameliorate nicotine-containing pharmaceutical formulations for oral delivery in order to avoid off-notes from the buffers used.

### Summary of the Invention

The present invention provides a buffered pharmaceutical oral formulation in the form of a mouth spray comprising nicotine, characterized in that the formulation is buffered with at least trometamol as the only buffer, or as the main buffer.

Also provided is an oral formulation according to the invention for use in obtaining a fast and/or sustained and/or complete reduction of the urge to smoke and use tobacco or for providing a sense of smoking without smoking.

Also provided is an oral formulation according to the invention for use in delivering nicotine to a subject.

Also provided is an oral formulation according to the invention for use in therapy.

The present invention also provides the use of nicotine for the production of an oral formulation according to the invention for the treatment of a disease selected from the group consisting of tobacco or nicotine dependence, Alzheimer's disease, Crohn's disease, Parkinson's disease, Tourette's syndrome, ulcerous colitis and post-smoking-cessation weight control.

When formulating a medical product intended to dissolve in the oral cavity the organoleptic characteristics are essential. In many cases there is a need to obtain optimal pH in the oral cavity to be able to achieve sufficient uptake of the active ingredient. By using a buffering agent in the product said pH can be adjusted. However, most commonly used buffering agents possess distinct off-notes. Therefore, one or more flavoring agents are usually added to the formulation to cover these off-notes. Moreover, flavoring agents are also used in the formulation to accomplish a product with pleasant taste. The possibility of using a buffering agent with no off-taste, facilitates the formulation work and reduces the complexity of the flavoring process. The buffering agent trometamol possesses no intrinsic taste and consequently, the use of trometamol in products for oral uptake has been found to be beneficial.

In view of the foregoing disadvantages known in the art when trying to deliver nicotine to a subject so as to obtain a transmucosal uptake of nicotine in the oral cavity of the subject the present invention provides a new and improved product for obtaining a transmucosal uptake of nicotine in the oral cavity of the subject, while avoiding off-notes from the buffer used.

An object of the present invention is to provide an efficient and effective product for uptake of nicotine in a subject and to avoid the disadvantages of such previously known products and methods.

Thus, the present invention provides a buffered pharmaceutical oral formulation in the form of a mouth spray comprising nicotine, characterized in that the formulation is buffered with at least trometamol as the only buffer, or as the main buffer.

Also described herein is a method for delivering nicotine in any form to a subject comprising administering to a subject an oral formulation containing nicotine in any form into the oral cavity of the subject and if needed allowing the nicotine in any form in the oral formulation to be released in the saliva in the oral cavity and absorbed into the systemic circulation of the subject as well as a method for producing said oral formulation. In a mouth spray the nicotine is directly available wherefore it need not be released as such in the saliva as said above. Hence the phrase "if needed" is inserted in the preceding sentence and in corresponding sentences below and in the claims.

Also described herein is a method for obtaining reduction of the urge to smoke or use tobacco containing material and/or for providing a sense of smoking satisfaction without smoking, comprising the steps of replacing at least partly the tobacco containing material with the above said oral formulation, administering to a subject an oral formulation containing nicotine in any form into the oral cavity of the subject and if needed allowing the nicotine in any form of the oral formulation to be released in the saliva in the oral cavity and absorbed by the subject.

Furthermore, also described is a system for delivering nicotine in any form to a subject, comprising said oral formulation and at least one other means for obtaining reduction of the urge to smoke or use of tobacco as well as a system for obtaining reduction of the urge to smoke or otherwise use tobacco and/or for providing a sense of smoking satisfaction without smoking, comprising an oral formulation as described above and at least one other method for obtaining reduction of the urge to smoke or otherwise use tobacco. Said system may be a system wherein the at least other method is selected from the group consisting of administration through mouth sprays, nasal sprays, transdermal patches, inhaling devices, lozenges, tablets and parenteral methods, subcutaneous methods, and transmucousal methods; or other use of tobacco.

By using trometamol as the only buffer, or as the main buffer, in said oral formulation the aforesaid problem with off-notes from the buffers used is solved.

Trometamol, chemically known as 2-amino-2-hydroxymethyl-1,3-propanediol, is also called tromethamine, tris(hydroxymethyl)aminomethane and TRIS. It is known as a "biological buffer" and as "alkalizer", see e g The Merck Index, 13^{th} Edition, 2001.

Nicotine-containing enemas comprising trometamol as buffer are known, see Italian Journal of Gastroenterology & Hepatology 30(3):260-5, 1998 June. Said enemas are intended for treating ulcerative colitis, i e for local treatment. This differs in essence from the present use, which is for systemic treatment and for a totally different use.

U.S. Patent Number 5,824,334 discloses trometamol as a buffer in a lollipop-like cigarette substitute. Lollipop-like devices are not primarily envisaged within the present invention.

US 5,783,207 discloses a lollipop-like device comprising a nicotine-containing matrix attached to a holder member so that nicotine may be administered to the oral cavity by sucking the matrix. The matrix may contain a buffer such as trometamol. '207 explicitly disclaims the use of chewing gums, tablets, lozenges and other dosage forms not attached to a holder member. This means that '207 teaches away from dosage forms not comprising a holder member. The present invention does not encompass lollipop-like dosage forms or other dosage forms comprising a holder member. All embodiments of the present invention are such that once they have been applied in the oral cavity they need not be further handled by any member from outside the oral cavity during the administration phase.

WO 01/30288 discloses in laundry lists nicotine and trometamol for use in formulations for oral mucosal delivery, such formulations having a dissolution agent with which e g the nicotine is in a specific type of solid solution. In the present invention nicotine is not in such solid solution.

The oral formulation according to the present invention is buffered with at least trometamol in such a way that upon administration of the formulation the pH of the saliva is increased by 0.2 - 4 pH units, or preferably increased by 0.5 - 2 pH units. The buffering agent trometamol may be supplemented with one or more buffers selected from the group consisting of a carbonate (including bicarbonate or sesquicarbonate), glycinate, phosphate, glycerophosphate or citrate of an alkali metal (such as potassium and sodium), e g trisodium and tripotassium citrate, or ammonium, and mixtures thereof. The main reason for supplementing trometamol with other buffers as above is to increase the buffering capacity per weight of the added buffers.

### Detailed Description of the Invention

### Definitions

As used herein, the term "oral formulation" or similar intends to mean all formulations being suitable to be placed in the oral cavity for delivering nicotine essentially to the tissue of the oral cavity. The oral formulation of the present invention is a mouth spray.

The term "intraoral delivery" is herein intended to mean delivery into the systemic blood circulation by means of absorption of an active principle by any tissue of the oral cavity.

The term "complete reduction" or "complete" is herein intended to mean complete or substantially complete reduction.

The term "controlled release" is intended to mean a release of nicotine from an oral formulation in the oral cavity of the subject, whereby active sucking or other manipulation of the oral formulation is controlling the amount of nicotine released.

The term "slow release" is intended to mean that nicotine is released from the oral formulation upon sucking or other manipulation over a period of time for example, several minutes to an hour.

The term "unit formula" is intended to mean one oral formulation unit.

The term "transient" is intended to mean a non-permanent change, upon which the relevant state, e g biological or physiological state, after a certain period of time will return to its value or behaviour prior to said change.

The terms "buccal" and "buccally" are herein intended to pertain to all of or any part of the tissue of the oral cavity.

### Useful oral formulations

Most dosage forms intended for oral delivery of nicotine benefit from using trometamol as the only or main buffering agent. These formulations include e g mouth sprays, chewing gums, tablets, melt tablets, lozenges, hard boiled candies, chewy candies, gummies, capsules, oral films, and liquid as well as powder formulations for intraoral and pulmonary inhalation.

The formulations of the invention are mouth sprays. These are discreet dosage forms being useful for obtaining a rapid uptake of nicotine through the mucosa of the oral cavity. Mouth sprays may in particular be sprayed under the tongue. Below Example 3 discloses the manufacturing of a mouth spray according to the invention.

The amount of gum base in a chewing gum as described herein is about 15 - 80 % by weight of the total gum core, and preferably about 40-80%. The amount of gum base employed for slow release of nicotine is usually in the higher ranges when nicotine is employed *per se* or when an absorbed form is used.

The gum base may be of any conventional nature known in the art. For example, it may comprise a gum base of natural or synthetic origin readily available from a commercial source. Natural gum bases include e g chicle, jelutong-, lechi de caspi-, soh-, siak-, katiau-, sorwa-, balata-, pendare-, malaya-, and peach gums, natural cautchouc and natural resins such as dammar and mastix. Synthetic gum bases are a mixture of:
- elastomers (for example polymers and/or masticating substances),
- plasticizers (for example resins, elastomers and/or solvents)
- fillers (for example texturizers and/or water-insoluble adjuvants),
- softeners (for example fats),
- emulsifiers,
- waxes,
- antioxidants,
- and anti-tacking agents (for example vinyl polymers and/or hydrophilic resins)

Other examples of gum bases are gums including agar, alginate, arabic gum, carob gum, carrageenan, ghatti gum, guar gum, karaya gum, pectin, tragacanth gum, locust beam gum, gellan gum and xanthan gum.

Examples of gelling agents comprise gum arabic, starch, gelatine, agar, and pectin.

When the nicotine in any form and the buffering agent or agents are incorporated in the chewing gum mass, it is possible to employ a wide variety of chewing gum compositions and amounts of the chewing gum base. Different chewing gum products may be composed depending on the consumers' preference and the purpose of use, in respect of the nicotine level, nicotine distribution and other additives.

Further below follow Examples on useful chewing gums, tablets, melt tablets, mouth sprays, soft capsules, hard boiled candies, oral films, gummies and chewy candies. On the basis of said Examples also other useful embodiments are envisageable.

### The buffering agent

Absorption of nicotine from the oral cavity to the systemic circulation is dependent on the pH of the saliva, pH of the blood plasma and the pKa of nicotine, which is about 7.8. Assuming a pH of the saliva of 6.8, only about 10% of the nicotine will be in the free base form. Thus, in order to promote absorption of nicotine in a free base form, which is the form predominantly absorbed through the mucosa, the pH of the saliva must be increased. At a pH of 8.8 about 90% of the nicotine will then be in the free base form.

Thus, according to the invention, the oral formulation is buffered with at least trometamol as the only buffer, or as the main buffer.

The buffering is designed so as to achieve a transient buffering of the saliva of a subject during melting, disintegration or dissolution of the oral formulation. As the change is transient, the pH will return to its normal value after a certain period of time.

By employing said change, here an increase, in said pH of the saliva the transmucosal uptake of nicotine in the oral cavity is changed, e g increased compared to the nicotine uptake when the saliva is not buffered according to the invention. Also, since the transmucosal uptake of nicotine in the oral cavity according to the invention is faster than for nicotine not being buffered according to the invention, less nicotine will be swallowed to reach the gastrointestinal (G.I.) tract. The nicotine that reaches the G.I. tract will be subjected to first pass metabolism which reduces the total amount of intact nicotine absorbed. This means that the bio-availability of nicotine that is not co-administered with a buffer will generally be lower than when administered together with a buffer.

Further embodiments of the invention includes oral dosage forms being buffered with trometamol in combination with other buffers, preferably selected from the group consisting of a carbonate including bicarbonate or sesquicarbonate, glycinate, phosphate, glycerophosphate or citrate of an alkali metal, such as potassium or sodium, or ammonium, and mixtures thereof.

Further embodiments may include combinations of trometamol with trisodium or tripotassium citrate, and mixtures thereof. Useful ratios between trometamol and such agents are provided in the below Examples.

Still further embodiments may encompass use of trometamol together with different phosphate systems, such as trisodium phosphate, disodium hydrogen phosphate; and tripotassium phosphate, dipotassium hydrogen phosphate, and calcium hydroxide, sodium glycinate; and mixtures thereof.

Alkali metal carbonates, glycinates and phosphates are preferred additional buffering agents.

In order to increase the buffering capacity still further without correspondingly increasing the pH, one may in specific embodiments use a second or auxiliary buffering agent to the first buffering agent, such as e g sodium or potassium bicarbonate buffers. Thereby should be strived to maintain a pleasant taste. The second or auxiliary buffering agent may be selected from the group consisting of alkali metal bicarbonates that are preferred for this purpose. Thus, further embodiments of the invention may comprise trometamol and a mixture of an alkali metal carbonate or phosphate and alkali metal bicarbonate. Useful mixture ratios are provided in the below Examples.

The amount of the buffering agent or agents in the oral formulation is preferably sufficient in the specific embodiments to raise the pH of the saliva to above 7, as specified above, to transiently maintain the pH of the saliva in the oral cavity above 7, e g pH 7 - 10.

The nicotine may be administered in different forms, e g in different complexes or salts. The amount of buffer required to achieve an increase in pH of the different administered nicotine forms is readily calculated by the skilled man in the art. The extent and duration of the increase in pH is dependent on type and amount of the buffering agent(s) used as well as where is further described within the paragraphs below.

### The active ingredient

According to the invention, the present oral formulation comprises nicotine in any form (for example free base, salt or complex).

With nicotine it is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination; or pharmaceutically acceptable salts.

The nicotine should be in a saliva soluble form to facilitate the release of the nicotine into the saliva in the oral cavity and, further, the subsequent uptake of the nicotine from the saliva in the oral cavity into the systemic circulation of the subject. When the nicotine prevails in the form of nicotine resinate complex, NRC, its solubility is modified in the presence of a buffer.

In preferred embodiments, the nicotine in any form is selected from the group consisting of the free base form of nicotine, a nicotine salt, a nicotine derivative, such as a nicotine cation exchanger, a nicotine inclusion complex or nicotine in any non-covalent binding, nicotine bound to zeolites, nicotine bound to cellulose or starch micro spheres, and mixtures thereof.

Numerous nicotine salts are known, and may be used, e g the salts presented in Table 1, preferably monotartrate, hydrogen tartrate (also called bitartrate or bitartrate dihydrate), citrate, malate, and/or hydrochloride.

**Table 1 Possible acids used for nicotine salt formation**

| Acid | Molar ratio* of acid:nicotine |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |
| * recommended at the time of production | |

The inclusion complex may be a cyclodextrin, such as β-cyclodextrin.

Suitable cation exchangers are given in below Table 2 and are further disclosed in U.S. Patent Number 3,845,217. Preferred are nicotine cation exchangers of polyacrylates, such as the Amberlite collection from Rohm & Haas.

**Table 2 Representative cation exchangers**

| Name | Type of crosslinked polymer | Manufacturer |
|---|---|---|
| Amberlite IRC 50 | Divinylbenzene-methacrylic acid | Rohm & Haas |
| Amberlite IRP 64 | Divinylbenzene-methacrylic acid | Rohm & Haas |
| Amberlite IRP 64M | Divinylbenzene-methacrylic acid | Rohm & Haas |
| BIO-REX 70 | Divinylbenzene-acrylic acid | BIO-RAD Lab. |
| Amberlite IR 118 | Styrene-divinylbenzene | Rohm & Haas |
| Amberlite IRP 69 | Styrene-divinylbenzene | Rohm & Haas |
| Amberlite IRP 69M | Styrene-divinylbenzene | Rohm & Haas |
| BIO-REX 40 | Phenolic | BIO-RAD Lab. |
| Amberlite IR 120 | Styrene-divinylbenzene | Rohm & Haas |
| Dowex 50 | Styrene-divinylbenzene | Dow Chemical |
| Dowex 50W | Styrene-divinylbenzene | Dow Chemical |
| Duolite C 25 | Styrene-divinylbenzene | Chemical Process Co |
| Lewatit S 100 | Styrene-divinylbenzene | Farbenfabriken Bayer |
| Ionac C 240 | Styrene-divinylbenzene | Ionac Chem |
| Wofatit KP S 200 | Styrene-divinylbenzene | I.G. Farben Wolfen |
| Amberlyst 15 | Styrene-divinylbenzene | Rohm & Haas |
| Duolite C-3 | Phenolic | Chemical Process |
| Duolite C-10 | Phenolic | Chemical Process |
| Lewatit KS | Phenolic | Farbenfabriken Bayer. |
| Zerolit 215 | Phenolic | The Permutit Co. |
| Duolite ES-62 | Styrene-divinylbenzene | Chemical Process |
| BIO-REX 63 | Styrene-divinylbenzene | BIO-RAD Lab. |
| Duolite ES-63 | Styrene-divinylbenzene | Chemical Process |
| Duolite ES-65 | Phenolic | Chemical Process |
| Ohelex 100 | Styrene-divinylbenzene | BIO-RAD Lab. |
| Dow Chelating Resin A-1 | Styrene-divinylbenzene | Dow Chemical Company |
| CM Sephadex C-25 | Dextran | Pharmacia Fine Chemicals |
| SE Sephadex C-25 | Dextran | Pharmacia Fine Chemicals |

One or more additives may be added to the present oral formulation. Additives are further described in the below paragraph *Other additives to the oral formulation.*

### Amount and distribution of the nicotine in the oral formulation

The nicotine in any form according to the invention is formulated to provide the subject with a dose to achieve an effect. The effect maybe to provide a sense of smoking satisfaction without smoking. Another effect of the administered nicotine in any form may be a reduction of the urge to smoke or use tobacco.

The effect may also be a combination of reduction of said urge and providing a sense of smoking satisfaction without smoking. The amount of the nicotine should be sufficient to provide such an effect in a subject. This amount may, of course, vary from person to person.

According to the invention, embodiments of the oral formulation comprise embodiments wherein nicotine in any form is present in an amount of 0.05 - 8 mg calculated as the free base form of nicotine per unit dose of the oral formulation. This may in different embodiments include 0.1, 0.5, 1, 2, 3, 4, 5, 6 or 8 mg calculated as the free base form of nicotine per unit dose.

Still preferred embodiments may contain embodiments where the nicotine in any form is present in an amount of 0.5 - 6 mg calculated as the free base form of nicotine per unit does of the oral formulation.

Even more preferred embodiments contain the nicotine in any form in an amount of 0.5 - 5 mg calculated as the free base form of nicotine per unit dose of the oral formulation.

The nicotine in any form may be distributed in the oral formulations in different embodiments. Different distributions of the nicotine throughout the oral formulations will imply administration of the nicotine to the subject in different ways. This may, then, provide several possibilities to adjust the composition of the oral formulation according to different needs of different subjects depending on the urge to smoke or use tobacco of the subject. In the below Examples are disclosed different such embodiments.

### Other additives to the oral formulation

Other additives may be added optionally to the oral formulation. Optional additives comprise at least one or more additives selected from the group consisting of solvents, such as ethanol and water; co-solvents, such as propylene glycol; stabilisers, such as preservatives, e g antioxidants; softeners, such as sorbitol and glycerine; thickening agents, such as colloidal silicon dioxide; binding agents, such as xanthan gum; filling agents, such as mannitol, isomalt, cocoa powder and Crospovidone; solubilizers, such as Polysorbat 80 and Atmos 300; rubbers, lipid barriers, such as sucrose fatty acid esters and hydrogenated vegetable oils; film forming agents, such as porcine gelatine, Pullulan, carrageenan, pectin, locust bean gum and xanthan gum; emulsifiers, such as pectin, soy lecithin, glycerol monostearate, castor oil and poloxamer; glidants, such as colloidal silicon dioxide; lubricants, such as magnesium stearate; coating agents, such as castor oil and sorbitol; melting vehicles, such as vegetable oils; sweeteners, flavors, aromatics, cooling agents, enhancers, colouring agents, vitamins, minerals, fluorine, breath fresheners, tooth whitening agents and mixtures thereof. According to the invention, at least one of such additives is optionally added to the product.

Enhancers may be added essentially to increase the transmucosal uptake of nicotine from the oral cavity.

Sweeteners are added essentially to improve the taste. Sweeteners comprise one or more synthetic or natural sugars, i e any form of carbohydrates suitable for use as sweetener, as well as so called artificial sweeteners such as saccarin, sodium saccarin, aspartame, e g NutraSweet®, acesulfame or Acesulfame K, potassium acesulfame, thaumatin, glycyrrhizin, sucralose, dihydrochalcone, alitame, miraculin, monellin, stevside and neotame.

Suitable sweeteners may be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, e g starch hydrolysates, containing a mixture of dextrose, maltose and a range of complex sugars, invert sugar syrup, e g sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

The flavor and aroma additives may comprise one or more synthetic or natural taste-masking, flavoring or aromatizing agents. Flavor and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavor of the fruit, e g strawberry, raspberry and black currant; artificial and natural flavors of brews and liquors, e g cognac, whisky, rum, gin, sherry, port, and wine; tobacco, coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds, nuts (e g peanuts, coconuts, hazelnuts, chestnuts, walnuts, colanuts), almonds, raisins; and powder, flour, or vegetable material parts including tobacco plant parts, e g genus Nicotiana, in amounts not contributing significantly to the level of nicotine, and ginger.

Colouring additives may be selected from dyes being approved as a food additive.

Stabilizing additives may be selected from the group consisting of antioxidants including vitamin E, i e tocopherole, ascorbic acid, sodium pyrosulfite, butylhydroxytoluene, butylated hydroxyanisole, edetic acid and edetate salts ; and preservatives including citric acid, tartaric acid, lactic acid, malic acid, acetic acid, benzoic acid, and sorbic acid. Preferred embodiments comprise an antioxidant as the stabiliser, and even more preferably the antioxidant vitamin E and/or butylated hydroxytoluene (BHT).

### Method for delivering nicotine in any form to a subject

The invention may be used to deliver nicotine to the subject (person) in a variety of ways. Accordingly also described herein is a method for delivering nicotine in any form to a subject comprising the steps of:
a) administering to a subject an oral formulation containing nicotine in any form according to the invention into the oral cavity of the subject, and
b) if needed allowing the nicotine in any form in the oral formulation to be released in the saliva in the oral cavity and absorbed into the blood plasma of the subject.

The method for delivering nicotine in any form may further comprise the steps of:
c) administering the nicotine in any form in a sustained way over a period of time to the subject. Such a time period may be at least 5, 10, 20, 30 or 40 minutes.

### Method for obtaining reduction of the urge to smoke or use of tobacco

Another feature of the invention is the ability to use the invention to reduce the urge to smoke. A method for obtaining reduction of the urge to smoke or use tobacco containing material and/or for providing a sense of smoking satisfaction without smoking comprises the steps of:
a) replacing at least partly the tobacco containing material with an oral nicotine-containing formulation,
b) administering to a subject an oral formulation containing nicotine in any form into the oral cavity of the subject, and
c) if needed allowing the nicotine in any form in the oral formulation coating to be released in the saliva in the oral cavity and absorbed by the subject.

The method described herein may further comprise the steps of administering the nicotine in any form in a sustained way over a period of time to the subject. The period of time may be at least 5, 10, 20, 30 or 40 minutes.

Also, the method for delivering nicotine to a subject may comprise the steps of combining administration of the oral formulation with at least one other method for obtaining reduction of the urge to smoke or use of tobacco.

Tobacco containing material may be material used for e g smoking, snuffing or chewing and may comprise a cigarette, a cigar, pipe tobacco, snuff, snus and chewing tobacco.

### Sustained reduction of the urge to smoke or use of tobacco

The invention may also be used to reduce the urge to smoke or use tobacco. Still, to continue the feeling or sense of satisfaction of the subject, and to avoid that the craving returns, a sustained craving relief may be obtained after the initial craving relief. A sustained craving relief is obtained by using the oral formulation in such a way as to allow a sustained uptake of the nicotine. The sustained craving relief and/or feeling or sense of satisfaction of the subject will continue as long as the subject maintains the blood plasma levels of nicotine at a level high enough to reach this sense of feeling.

The subject may achieve this by using the oral formulation over a period of time, such as 5, 10, 20, 30 or 40 minutes or longer, e g a slow release of the nicotine caused by a controlled release, e g by individual use.

### Cessation of the urge to smoke or use of tobacco

For some of the users, it may be a goal to terminate the usage of nicotine completely, due to several reasons e g health, economical, social or behavioural. This cessation of smoking or the urge to use tobacco may be achieved by further decreasing the amount of nicotine in any form gradually over time. The method described above for obtaining craving relief may further comprise the steps of decreasing the amount of nicotine in the oral formulation described above gradually over time, so as to achieve a complete relief of tobacco craving. This method results in a weaning process gradually over time.

Different types of smokers reach the sense of reduced craving at different plasma levels of nicotine. This may, of course, affect the individual types of administration programs of an oral formulation according to the invention. Different types of smokers include e g peak seekers or smokers that crave for a plasma level of nicotine constantly being above the level for withdrawal symptoms.

One strategy may be to lower the frequency of the administered oral formulation. Other embodiments include varying the dose of the nicotine in said oral formulations as well as the combination of these two. Also, the strategy may include an oral formulation with substantially no nicotine in any form. Such an oral formulation may be administered at the end of the treatment period, when the craving is low or substantially absent.

### Systems for delivering nicotine and for obtaining craving relief

Also described is a system for delivering nicotine in any form to a subject particularly for obtaining craving relief. Such a system comprises an oral formulation according to the invention and at least one other means for obtaining reduction of the urge to smoke.

Another system may also be a system for obtaining reduction of the urge to smoke or use of tobacco and/or for providing a sense of smoking satisfaction without smoking. Such a system comprises an oral formulation according to the invention and at least one other method or means for obtaining reduction of the urge to smoke or use tobacco. Other methods and means may also be a concomitant or concurrent method selected from the group consisting of administration through mouth sprays, nasal sprays, transdermal patches, inhaling devices, lozenges, tablets and parenteral methods, subcutaneous methods, and transmucosal methods; or use of tobacco.

Specifically, the at least other method may comprise administration of nicotine.

### Use of the oval formulation

The use of the oral formulation according to the invention may include obtaining a fast and/or sustained and/or complete reduction of the urge to smoke and use tobacco or for providing a sense of smoking without smoking as described above.

The dose of the nicotine is chosen to give the subject an individual sensory perception and satisfaction with an effect of the nicotine in any form. The use of an oral formulation may also be a sole use according to the invention or a combination with other means or methods known in the field of drug abuse. Specifically, the present invention may be used in combination with other means as described above in the methods in the paragraphs above.

The use may give a quick reduction of the urge to smoke or use tobacco.

Other embodiments will imply a use giving a slow reduction of the urge to smoke or use tobacco.

### Use for therapy and treatment

The oral formulation according to the invention may be used in therapy and treatment. Said therapy may be a treatment of a disease selected from the group consisting of tobacco or nicotine dependence, Alzheimer's disease, Crohn's disease, Parkinson's disease, Tourette's syndrome, ulcerous colitis and post-smoking-cessation weight control.

Nicotine may also be used for an oral formulation according to the invention for the treatment of said diseases.

Further, nicotine may be used in the production of a nicotine-containing oral formulation according to the invention for the treatment of said diseases.

### Production of the oval formulation

The oral formulations according to the present invention are basically produced according to methods known in the art. Exemplary, but not limiting, production methods are provided below under Examples.

In the below Examples is described the mixing, rolling and scoring as well as the compression of chewing gums.

The below Examples also provide information on manufacturing of other embodiments described herein.

Conveniently, the compositions of additives according to the invention, e g the buffer system, are made simultaneously, according to known procedures in the art for formulating e g the buffers. Depending on the physical properties of the buffer system incorporated, it may be convenient to add the buffer system/s either with the liquid part or with the solid part of the composition. In the case of buffering systems available as fine powders, it may, of course, be most convenient to add those powders with the solid, powdered part of other additives.

The final product may then be analysed and further wrapped.

### Analysis of nicotine

The analysis of nicotine uptake and effect according to the invention may be done according to standard procedures known in the art, e g using bioanalysis for the determination of nicotine or its metabolites in the plasma of a subject.

### Examples

The below examples are illustrative and non-limiting. The skilled person may on the basis of the following examples envisage also other embodiments of the present invention. Batch sizes for the manufacture of the below formulations may be modified according to the actual need and to the actual production facilities. If not stated otherwise procedures and equipment known in the art are used in the below manufacturing.

### Reference Example 1 Tablets

**275 mg tablet with 2 mg nicotine**

| Ingredients | Amount in composition (mg) |
|---|---|
| Nicotine bitartrate dehydrate | 6,1 |
| Trometamol | 12,4 |
| Mannitol | 216,4 |
| Xanthan gum | 11,0 |
| Crospovidone | 11,0 |
| Flavoring agents | 9,9 |
| Aspartame | 1,6 |
| Acesulfame K | 1,1 |
| Magnesium stearate | 5,5 |

### Manufacturing process:

The above ingredients are blended. The blend is then compressed into tablets by means of direct compression according to methods known in the art.

### Reference Example 2 Melt tablets

This is a tablet intended for melting in the mouth whereupon the melted material adheres to the oral mucosa where the nicotine is deposited for entering into the tissue.

**400 mg melt tablet with 2 mg nicotine**

| Ingredients | Relative amount in composition (%w/w) |
|---|---|
| Nicotine bitartrate dehydrate | 1.5 |
| Cocoa powder | 35.0 |
| Vegetable oil | 41.6 |
| Trometamol | 3.1 |
| Mannitol | 11.8 |
| Titanium dioxide | 2.7 |
| Soy lecithin | 1.0 |
| Aspartame | 0.4 |
| Acesulfame K | 0.2 |
| Flavoring agents | 2.7 |

### Manufacturing process:

The manufacturing as such takes place at room temperature. A part of the fatty component, i e the vegetable oil, is melted. The solid components, i e the nicotine salt, the cocoa powder, the buffering agent, the mannitol, the titatnium oxide, the sweeteners and the flavoring agents are added and mixed. A reduction of particle size of the solid components is performed by milling the mixture in a roll-refiner. If the solid components have already got the required particle size, e g by milling before the mixing with the fatty component, roll refining is dispensed with. After possible treatment in the roll-refiner the mixture is mixed with the rest of the melted vegetable oil or remelted (if solidified) and mixed with the rest of the melted vegetable oil. A mixing of the melt is performed in a suitable mixer. The liquid component, i e the soy lecithin, is added.

Tablets are subsequently made using suitable techniques, such as molding, extrusion or congealing, including pastillation, when necessary after suitable preconditioning. Also other suitable manufacturing methods known in the art may be used.

### Example 3 Mouth sprays

**Nicotine mouth spray with 14.3 mg nicotine/ml and pH 9.0**

| Ingredients | mg/ml |
|---|---|
| Nicotine free base | 14.3 |
| Ethanol | 100.0 |
| Propylene glycol | 150.0 |
| Glycerine | 25.0 |
| Trometamol | 40.5 |
| Sodium Hydrogen Carbonate | 14.3 |
| Poloxamer | 40.0 |
| Levomenthol | 10.0 |
| Flavoring agent | 4.0 |
| Cooler | 3.0 |
| Sweeteners | 3.0 |
| Hydrochloric acid | Ad pH 9.0 |
| Purified water | q.s. |

### Manufacturing process:

1. Ethanol is charged into a vessel.
2. The flavoring agents and poloxamer are added. The components are dissolved during mixing.
3. Purified water is gently added while stirring.
4. Tetracemindinatrium, trometamol, sweeteners are added and mixing is continued.
5. Nicotine is added to the solution while gently stirring.
6. pH of the solution is measured. When needed, the pH is adjusted to 9.0 by adding hydrochloric acid 1M.
7. Purified water is added q.s. to batch quantity. The solution is mixed until a clear solution is obtained.

### Reference Example 4 Capsules

**Nicotine soft capsules 2 mg**

| Ingredients | % (w/w) |
|---|---|
| *Ingredients of Core:* | |
| Nicotine free base | 2.2 % |
| Medium chain triglycerides | 87.5 % |
| Flavors and sweeteners | 7.8 % |
| Trometamol | 2.0 % |
| Colloidal silicon dioxide | 0.5 % |

| *Ingredients of Inner Shell:* | |
|---|---|
| Sucrose fatty acid ester | 60.0 % |
| Hydrogenated vegetable oil | 40.0 % |

| *Ingredients of Outer Shell:* | |
|---|---|
| Porcine gelatin | 80.0 % |
| Sorbitol | 18.0% |
| Glycerin | 2.0 % |

| *Weight Ratio:* | |
|---|---|
| *Core*/*Inner shell*/*Outer shell* | 64/30/6 |
| *Total Capsule weight:* | 142 mg |

### Use:

Seamless soft gel capsules are soft gelatin capsules that are distinguished by their spherical shape and thin, seamless gelatin shell. The thin shell makes the capsules suitable for use in orally dissolving products compared to conventionally produced soft gelatin capsules that are intended to be chewed or swallowed.

### Manufacturing process:

Seamless soft gel capsules are manufactured by formation of droplets consisting of two or more concentric layers. The droplets are formed by feeding different liquids through concentric nozzles. The outermost nozzle feeds a hydrophilic solution consisting of gelatin and additives e g plasticizers. The one or more inner nozzles feed a lipophilic liquid (e g oils, triglycerids) wherein one or more active substances may be dispersed. The lipophilic centre and hydrophilic perimeter of the formed droplets ensure a good phase separation between shell and core contents. The formed capsules are then subjected to sequential processing steps such as cooling, drying, washing and selection of size and shape.

### Reference Example 5 Hard boiled candies

**Nicotine hard boiled candy with 2 mg nicotine**

| Ingredients | % (w/w) |
|---|---|
| Purified water | - |
| Isomalt | 78.5 |
| Maltitol 75 % solution | 19.5 |
| Nicotine bitartrate dihydrate | 0.2 |
| Trometamol | 1.1 |
| Flavor | 0.7 |
| | |
| Total | 100.0 |
| Piece weight 3.5 g | |
| Nicotine /piece 2 mg | |

### Manufacturing process:

1. To a stainless steel beaker add purified water, isomalt and maltitol solution. Mix and heat during continuous mixing.
2. Discontinue heating and cool to 135-140 °C. Add nicotine bitartrate dihydrate and mix until fully dispersed. Add trometamol and mix at 120 °C until dispersed.
3. Add flavor, mix until uniform.
4. Pour into molds, let cool.

### Reference Example 6 Oral films

**Nicotine bi-layer film with 2 mg nicotine**

| Ingredient | mg/film (Active part) | mg/film (Buffer part) |
|---|---|---|
| Nicotine free base | 2.00 | - |
| Pullulan | 30.0 | 30.0 |
| Xanthan gum | 0.04 | 0.04 |
| Locust bean gum | 0.08 | 0.08 |
| Carrageenan | 0.38 | 0.38 |
| Pectin | 0.27 | 0.27 |
| Polysorbat 80 | 0.46 | 0.46 |
| Atmos 300 | 0.46 | 0.46 |
| Sucralose | 2.10 | 2.10 |
| Sorbitol | 2.90 | 2.90 |
| Menthol | 1.95 | 1.95 |
| Flavor | 12.7 | 12.7 |
| Colouring agent | 0.02 | 0.02 |
| Trometamol | - | 2.84 |
| Sodium carbonate anhydrous | - | 1.23 |
| Tartaric acid | 3.7 | - |
| Purified water | About 4.20 | About 4.20 |
| Sub-Total | 60 | 60 |
| Total | 120 | |

### Manufacturing process (Part 1)

1. Mix together Pullulan, xanthan gum, locust bean gum, carrageenan and pectin.
2. Add heated water to the mixture
3. Add sucralose and sorbitol, mix to dissolve. Cool to room temperature.
4. Pre-mix Polysorbate 80, Atmos 300, colouring agent, menthol and flavor, and add to the blend.
5. Add tartaric acid and then nicotine free base and mix.
6. Cast Pullulan solution onto substrate of desired thickness and dry with hot air.

### Manufacturing process (Part 2):

1. Mix together Pullulan, xanthan gum, locust bean gum, carrageenan, pectin, trometamol and sodium carbonate.
2. Add heated water to the mixture.
3. Add sucralose and sorbitol, mix to dissolve. Cool to room temperature.
4. Pre-mix Polysorbate 80, Atmos 300, colouring agent, menthol and flavor, and add to the blend
5. Cast Pullulan solution onto substrate of desired thickness and dry with hot air.

### Manufacturing (Part 3)

1. Casted film (Active) and casted film (Buffer) are laid upon each other and slightly pressed together.
2. Cut into desired size. Size of e g 24 mm x 33 mm is appropriate.

If necessary a barrier layer may be placed between the casted active-containing film and the casted buffer-containing film in order to avoid chemical reactions between these two films.

Also mono-layer and multi-layer oral films are envisageable.

### Reference Example 7 Gummies

**Nicotine gummy with 1 mg nicotine**

| Ingredients | g per piece |
|---|---|
| Isomalt | 3.7 |
| Sweetener | 1.0 |
| Water | 0.1 |
| Pectin | 0.1 |
| Trometamol | 0.025 |
| Flavor | 0.1 |
| Nicotine bitartrate dihydrate | 0.0032 |
| Total | 5 g |

### Manufacturing process:

1. Heat the isomalt to melting point and add sweetener and let the mixture cool.
2. To the cooled mixture, add pectin solution, trometamol and flavor.
3. Add nicotine bitartrate dihydrate, mix thoroughly.
4. Cast using starch moulds in desired shape and size using methods known in the art.

### Reference Example 8 Chewy candies

**Nicotine chewy candy with 1 mg nicotine**

| Ingredients | g per piece |
|---|---|
| Isomalt | 3.4 |
| Sweetener | 1.0 |
| Water | 0.1 |
| Vegetable oil | 0.3 |
| Glycerol monostearate | 0.1 |
| Trometamol | 0.025 |
| Flavor | 0.1 |
| Nicotine bitartrate dihydrate | 0.0032 |
| Total | 5 g |

### Manufacturing process

1. Heat the isomalt to melting point and add sweetener and let the mixture cool.
2. To the cooled mixture, add vegetable oil, trometamol and flavor. Mix well.
3. Add nicotine bitartrate dihydrate. Mix well. Cast in molds or extrude and cut to desired size using methods known in the art.

### Reference Example 9 Compressed chewing gums

### Reference Example 9A

**Nicotine-containing compressed chewing gum with 2 mg nicotine**

| Ingredients | Amount in composition (mg) |
|---|---|
| Nicotine resin complex (NRC) 20% | 10 |
| Chewing gum base | 556 |
| Trometamol | 25 |
| Sodium carbonate | 10 |
| Castor oil | 60 |
| Sorbitol | 140 |
| Flavoring agents | 129 |
| Sweeteners | 5 |
| Colloidal silicon dioxide | 22.5 |
| Magnesium stearate | 20 |
| Talc | 22.5 |

### Manufacturing process:

1) The nicotine resin complex is blended with the hydrophilic aqueous soluble element, i e sorbitol.
2) The hydrophobic element, which is practically insoluble in water, i e castor oil, is heated to a suitable temperature until a solution is obtained.
3) The blend obtained in 1) is added to the solution 2) under vigorous stirring.
4) The above blend in 3) is cooled to below room temperature and blended with gum base and other additives.
5) The above blend in 4) is, if necessary, sieved to remove aggregates and compressed into gums by means of direct compression.

### Reference Example 9B

**Nicotine compressed chewing gum with 2 mg nicotine**

| Ingredients | Amount in composition (mg) |
|---|---|
| Nicotine resin complex 20% | 10 |
| Chewing gum base | 300 |
| Trometamol | 25 |
| Sodium carbonate | 10 |
| Isomalt | 100 |
| Sorbitol | 497 |
| Flavoring agents | 30 |
| Sweeteners | 3 |
| Colloidal silicon dioxide | 5 |
| Magnesium stearate | 20 |

### Manufacturing process description:

1) Mixing: A gum base powder mixture comprising gum base, sweeteners and glidant is blended with active, flavoring agent, glidant, artificial sweeteners, buffering agents and lubricant.
2) Tableting: The above blend is sieved to remove aggregates if necessary and compressed into gums by means of direct compression.

### Reference Example 10 Chewing gums made by mixing, rolling and scoring

| | 2 mg Unit formula (mg) | 4 mg Unit formula (mg) |
|---|---|---|
| Nicotine resin complex 20% | 10 | 20 |
| Chewing gum base | 660 | 660 |
| Xylitol | 260 | 240 |
| Trometamol | 30 | 45 |
| Flavoring agents | 32 | 22 |
| Levomenthol | 2 | 2 |
| Magnesium oxide | 1 | 1 |

### Manufacturing process:

Mixing, rolling and scoring is done by a conventional procedure. Double sigma blade mixers are used for mixing the gum base with the other components of the formulation. The gum base is softened in the mixer. By heat (from the heating jacket) and mixing, the gum base becomes plastic. So, the softened base is mixed with the liquid components and the solid materials as a powder mixture. The warm mass is discharged from the mixer in form of loaves stacked on trays on a truck and stored in a conditioned area until the next step starts. This is to cool the gum

After this, the rolling and scoring takes place. The gum is extruded into a thick sheet, which is rolled by multiple sets of calender rolls to the correct thickness. The scoring rolls, usually two sets, cut the sheet into correctly sized pieces.

The sheets are then transferred to a conditioned area on trays, where the sheets are cooled to make them brittle enough to be broken. The conditioned gum sheets are then passed through a breaker, which is a rotating drum that parts the sheets into separate pieces of gum along the scores.

At a sorting stage deformed gums are sorted away. The accepted gums are passed through a metal detector.

## Claims

1. A buffered pharmaceutical oral formulation in the form of a mouth spray comprising nicotine, **characterized in that** the formulation is buffered with at least trometamol as the only buffer, or as the main buffer.

2. The oral formulation according to claim 1 being buffered with trometamol together with a buffer selected from the group consisting of a carbonate, such as bicarbonate or sesquicarbonate, glycinate, phosphate, glycerophosphate, citrate of an alkali metal, such potassium or sodium, or ammonium, and mixtures thereof.

3. The oral formulation according to claim 1 or claim 2, wherein the nicotine in any form is selected from the group consisting of a nicotine salt; the free base form of nicotine; a nicotine, derivative, such as a nicotine cation exchanger; a nicotine inclusion complex; or nicotine in any non-covalent binding; nicotine bound to zeolites; nicotine bound to cellulose or starch micro spheres; and mixtures thereof.

4. The oral formulation according to any of claims 1 - 3, wherein the nicotine in any form is present in an amount of 0.05 - 8 mg calculated as the free base form of nicotine per unit dose of the oral formulation.

5. The oral formulation according to any of claims 1 - 4, comprising at least one or more additive selected from the group consisting of solvents, co-solvents, stabilisers, preservatives, antioxidants, softeners, thickening agents, binding agents, filling agents, solubilizers, rubbers, lipid barriers, film forming agents, emulsifiers, glidants, lubricants, coating agents, melting vehicles, sweeteners, flavors, aromatics, cooling agents, enhancers, colouring agents, vitamins, minerals, fluorine, breath fresheners, tooth whitening agents and mixtures thereof.

6. An oral formulation according to claim 1 - 5 for use in obtaining a fast and/or sustained and/or complete reduction of the urge to smoke and use tobacco or for providing a sense of smoking without smoking.

7. An oral formulation according to any of claims 1 - 5 for use in delivering nicotine to a subject.

8. A formulation according to any one of claims 1-5, wherein the formulation is a nicotine-containing mouth spray comprising nicotine free base, ethanol or another solvent, trometamol, poloxamer or another solubilizer, tetracemindinatrium or another stabilizer, and artificial sweeteners.

9. A formulation according to claim 8, wherein trometamol is exchanged for trometamol in combination with a buffer selected from the group consisting of a carbonate including bicarbonate or sesquicarbonate, glycinate, phosphate, glycerophosphate or citrate of an alkali metal, such as potassium or sodium, or ammonium, including trisodium phosphate, disodium hydrogen phosphate; tripotassium phosphate, dipotassium hydrogen phosphate, and calcium hydroxide, sodium glycinatc; and mixtures thereof

10. A formulatior according to any of claims 1 - 5 or claim 8 for use in therapy.

11. A formulation according to claim 10, wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

12. Use of nicotine for the production of a product according to any of claims 1 - 5 or claim 8 for the treatment of a disease selected from the group consisting of tobacco or nicotine dependence, Alzheimer's disease, Crohn's disease, Parkinson's disease, Tourette's syndrome, ulcerous colitis and post-smoking-cessation weight control.

13. Use of nicotine for the production of an oral formulation according to any of claims 1 - 5 or claim 8 for the treatment of a disease selected from the group consisting of tobacco or nicotine dependence, Alzheimer's disease, Crohn's disease, Parkinson's disease, Tourette's syndrome, ulcerous colitis and post-smoking-cessation weight control.

## Patentansprüche

1. Gepufferte pharmazeutische orale Formulierung in Form eines Mundsprays, die Nikotin umfasst, **dadurch gekennzeichnet, dass** die Formulierung mit mindestens Trometamol als einzigem Puffer, oder als Hauptpuffer, gepuffert ist.

2. Orale Formulierung nach Anspruch 1, die mit Trometamol zusammen mit einem Puffer ausgewählt aus der Gruppe bestehend aus einem Carbonat, wie Bicarbonat oder Sesquicarbonat, Glycinat, Phosphat, Glycerophosphat, Citrat eines Alkalimetalls, wie Kalium oder Natrium, oder Ammonium und Mischungen davon gepuffert ist.

3. Orale Formulierung nach Anspruch 1 oder 2, wobei das Nikotin in jeder Form ausgewählt ist aus der Gruppe bestehend aus einem Nikotinsalz; der freien Grundform von Nikotin; einem Nikotinderivat, wie einem Nikotin-Kationenaustauscher; einem Nikotininklusionskomplex; oder Nikotin in irgendeiner nicht-kovalenten Verbindung; an Zeolithe gebundenem Nikotin; an Zellulose oder Stärkemikrosphären gebundenem Nikotin; und Mischungen davon.

4. Orale Formulierung nach einem der Ansprüche 1-3, wobei das Nikotin in jeder Form in einer Menge von 0,05 - 8 mg berechnet als freie Grundform von Nikotin pro Einheitsdosis der oralen Formulierung vorhanden ist.

5. Orale Formulierung nach einem der Ansprüche 1-4, die mindestens einen oder mehrere Zusatzstoffe ausgewählt aus der Gruppe bestehend aus Lösemitteln, Co-Lösemitteln, Stabilisatoren, Konservierungsstoffen, Antioxidantien, Weichmachern, Verdickungsmitteln, Bindemitteln, Füllstoffen, Löslichmachern, Gummis, Lipidbarrieren, Filmbildungsmitteln, Emulgatoren, Gleitmitteln, Schmiermitteln, Beschichtungsmitteln, Schmelzstoffen, Süßungsmitteln, Geschmacksstoffen, Aromastoffen, Kühlmitteln, Verstärkungsmitteln, Farbstoffen, Vitaminen, Mineralien, Fluor, Atemerfrischern, Zahnaufhellungsmitteln und Mischungen davon umfasst.

6. Orale Formulierung nach Anspruch 1-5 zum Gebrauch beim Erhalt einer schnellen und/oder nachhaltigen und/oder vollständigen Reduzierung des Dranges, zu rauchen und Tabak zu verwenden, oder zum Vermitteln eines Rauchgefühls, ohne zu rauchen.

7. Orale Formulierung nach einem der Ansprüche 1-5 zum Gebrauch bei der Abgabe von Nikotin an eine Person.

8. Formulierung nach einem der Ansprüche 1-5, wobei die Formulierung ein nikotinhaltiges Mundspray ist, das nikotinfreie Basis, Ethanol oder ein anderes Lösemittel, Trometamol, Poloxamer oder einen anderen Löslichmacher, Tetracemindinatrium oder einen anderen Stabilisator, und künstliche Süßungsmittel umfasst.

9. Formulierung nach Anspruch 8, wobei Trometamol ersetzt wird durch Trometamol in Verbindung mit einem Puffer ausgewählt aus der Gruppe bestehend aus einem Carbonat einschließlich Bicarbonat oder Sesquicarbonat, Glycinat, Phosphat, Glycerophosphat oder Citrat eines Alkalimetalls, wie Kalium oder Natrium, oder Ammonium, einschließlich Trinatriumphosphat, Dinatriumhydrogenphosphat, Trikaliumphosphat, Dikaliumhydrogenphosphat, und Kalziumhydroxid, Natriumglycinat und Mischungen davon.

10. Formulierung nach einem der Ansprüche 1-5 oder Anspruch 8 zum Gebrauch in der Therapie.

11. Formulierung nach Anspruch 10, wobei die Therapie eine Behandlung einer Krankheit ausgewählt aus der Gruppe bestehend aus Tabak- oder Nikotinabhängigkeit, Alzheimer, Morbus Crohn, Parkinson, Tourette-Syndrom, Colitis Ulcerosa und Gewichtskontrolle nach Raucherentwöhnung ist.

12. Gebrauch von Nikotin zur Herstellung eines Produkts nach einem der Ansprüche 1-5 oder Anspruch 8 zur Behandlung einer Krankheit ausgewählt aus der Gruppe bestehend aus Tabak- oder Nikotinabhängigkeit, Alzheimer, Morbus Crohn, Parkinson, Tourette-Syndrom, Colitis Ulcerosa und Gewichtskontrolle nach Raucherentwöhnung.

13. Gebrauch von Nikotin zur Herstellung einer oralen Formulierung nach einem der Ansprüche 1-5 oder Anspruch 8 zur Behandlung einer Krankheit ausgewählt aus der Gruppe bestehend aus Tabak- oder Nikotinabhängigkeit, Alzheimer, Morbus Crohn, Parkinson, Tourette-Syndrom, Colitis Ulcerosa und Gewichtskontrolle nach Raucherentwöhnung.

## Revendications

1. Formule pharmaceutique orale tamponnée sous la forme d'un spray buccal comprenant de la nicotine, **caractérisée en ce que** la formule est tamponnée par au moins du trométamol en tant que seul tampon, ou en tant que tampon principal.

2. Formule orale selon la revendication 1, qui est tamponnée par du trométamol et avec un tampon choisi dans le groupe constitué par un carbonate, tel que bicarbonate ou sesquicarbonate, glycinate, phosphate, glycérophosphate, citrate d'un métal alcalin, tel que potassium ou sodium, ou ammonium, et leurs mélanges.

3. Formule orale selon la revendication 1 ou la revendication 2, où la nicotine sous n'importe quelle forme est choisie dans le groupe constitué par un sel de nicotine ; la forme base libre de la nicotine ; un dérivé de nicotine, tel qu'un échangeur de cations de nicotine ; un complexe d'inclusion de nicotine ; ou de la nicotine dans n'importe quel environnement de liaison non covalente ; de la nicotine liée à des zéolithes ; de la nicotine liée à des microsphères de cellulose ou d'amidon ; et leurs mélanges.

4. Formule orale selon l'une quelconque des revendications 1 à 3, où la nicotine sous n'importe quelle forme est présente à une teneur de 0,05 à 8 mg, calculée en tant que forme base libre de nicotine par dose unitaire de la formule orale.

5. Formule orale selon l'une quelconque des revendications 1 à 4, comprenant au moins un ou plusieurs adjuvants choisis dans le groupe constitué par les suivants : solvants, cosolvants, agents stabilisants, conservateurs, antioxydants, adoucissants, agents épaississants, agents de liaison, charges, solubilisants, caoutchoucs, barrières lipidiques, agents filmogènes, émulsifiants, agents glissants, lubrifiants, agents d'enrobage, vecteurs fusibles, agents sucrants, arômes, agents aromatiques, agents de refroidissement, agents d'amplification, agents colorants, vitamines, minéraux, fluor, agents de rafraîchissement de l'haleine, agents de blanchiment dentaire et leurs mélanges.

6. Formule orale selon les revendications 1 à 5 pour utilisation dans l'obtention d'une réduction rapide et/ou prolongée et/ou totale de la pulsion de fumer et d'utiliser du tabac ou pour fournir une sensation de consommation de cigarettes sans consommation de cigarettes.

7. Formule orale selon l'une quelconque des revendications 1 à 5 pour utilisation dans la libération de nicotine à un sujet.

8. Formule selon l'une quelconque des revendications 1 à 5, où la formule est un spray buccal contenant de la nicotine comprenant la base libre de nicotine, l'éthanol ou un autre solvant, le trométamol, un poloxamère ou un autre agent solubilisant, du tétracémindinatrium ou un autre agent stabilisant, et les agents sucrants artificiels.

9. Formule selon la revendication 8, où le trométamol est remplacé par le trométamol en combinaison avec un tampon choisi dans le groupe constitué par un carbonate incluant bicarbonate ou sesquicarbonate, glycinate, phosphate, glycérophosphate ou citrate d'un métal alcalin, tel que potassium ou sodium, ou ammonium, incluant phosphate de trisodium, hydrogénophosphate de disodium ; phosphate de tripotassium, hydrogénophosphate de dipotassium, et hydroxyde de calcium, glycinate de sodium ; et leurs mélanges.

10. Formule selon l'une quelconque des revendications 1 à 5 ou selon la revendication 8 pour utilisation en thérapie.

11. Formule selon la revendication 10, où la thérapie est le traitement d'une maladie choisie dans le groupe constitué par les suivantes : dépendance au tabac ou à la nicotine, maladie d'Alzheimer, maladie de Crohn, maladie de Parkinson, syndrome de Gilles de la Tourette, rectocolite hémorragique et régulation du poids après arrêt de la cigarette.

12. Utilisation de la nicotine dans la production d'un produit selon l'une quelconque des revendications 1 à 5 ou la revendication 8, pour le traitement d'une maladie choisie dans le groupe constitué par les suivantes : dépendance au tabac ou à la nicotine, maladie d'Alzheimer, maladie de Crohn, maladie de Parkinson, syndrome de Gilles de la Tourette, rectocolite hémorragique et régulation du poids après arrêt de la cigarette.

13. Utilisation de la nicotine dans la production d'une formule orale selon l'une quelconque des revendications 1 à 5 ou la revendication 8, pour le traitement d'une maladie choisie dans le groupe constitué par les suivantes : dépendance au tabac ou à la nicotine, maladie d'Alzheimer, maladie de Crohn, maladie de Parkinson, syndrome de Gilles de la Tourette, rectocolite hémorragique et régulation du poids après arrêt de la cigarette.
